# EUROPEAN PATENT SPECIFICATION

(11) **EP 0 427 726 B1**
(45) Date of publication and mention of the grant of the patent: **28.07.1993**
(21) Application number: 89904627.0
(22) Date of filing: 13.04.1989
(51) Int. Cl.: G07F 7/06

(54) **AUTOMAT FOR ONE-TIME SYRINGES**
AUTOMAT FÜR WEGWERFSPRITZEN
AUTOMATE POUR SERINGUES A USAGE UNIQUE

(30) Priority: 15.04.1988 NO 881632
(43) Date of publication of application: 22.05.1991
(73) Proprietor: VEIDUNG, Arne, N-5062 Bones (NO)
(72) Inventor: VEIDUNG, Arne, N-5062 Bones (NO)
(74) Representative: Rostovanyi, Peter
(86) International application number: NO8900031
(87) International publication number: WO8909982

(56) References cited:
- FR-A- 2 506 479
- FR-A- 2 554 263
- FR-A- 2 602 355
- US-A- 4 411 351

## Description

The present invention relates to an automat for one-time syringes, where a number of syringes are stored in a storage space in the automat and can be dispensed individually via an outlet in the automat on actuation of an actuating means.

It has become an increasing problem that dangerously infectious substances (blood materials) can be transmitted via syringes from person to person. In this connection one-time syringes are a possibility for avoiding repeated use from user to user. However there is also a danger of infection being transmittable by unexpected contact with an infection-carrying syringe and there is a need to be able to take care of used syringes in an effective manner after use.

The aim of the invention is a solution where an unused syringe can be given up (free) from an automat on inserting a used syringe into the automat. However great weight is given to the fact, that if an unused syringe is to be given up on inserting a used syringe into the automat, one must be certain that it is a syringe which is inserted. In this connection the objective is to avoid giving up an unused syringe after inserting an imitation object or after other "tampering" of or misuse of the automat. Parallel with or in addition to such (free) giving up of new unused syringes in exchange for a used syringe there can be employed a principle known per se for liberating new, unused syringes based on inserting coins. At the same time the aim is to be able to collect and to take care in a secure manner of the used syringes, so that the transmission of infection is counteracted to the greatest degree possible, simultaneously with the aim to replace a used syringe with an unused syringe.

The present invention is characterised in that the automat comprises a collection container for receiving used syringes, and that an intake for used syringes communicates with the collection container which is different from end separated from the storage space for unusued syringes via a connecting duct which includes the actuating means or a special actuating means for sensing a used syringe and for activating the dispensing of an unused syringe via a separate outlet as a reaction to the insertion of the used syringe in said connecting duct.

It is advantageous that the connecting duct includes a first stop means, for intermittently shutting off the used syringe in a sensing position until the used syringe is discharged further into the collection container, the stop means on actuation by an inserted, used syringe being adapted to activate a second stop means for dispensing an unused syringe from the storage space, as a reaction to the actuation of the first stop means. It is a particular advantage that the connecting duct includes a sensing means, for sensing a used syringe in a specific sensing position in the connecting duct, a positive sensing of a used syringe in the connecting duct releasing a signal for activating a dispensing of an unused syringe.

In practice it is preferred that the first stop means comprises a flap with a normal initial position for shutting off an inserted used syringe in a sensing position in the connecting duct, and that the sensing means comprises a photocell which is arranged directly on or upstream immediately in front of the flap, the flap being adapted to be pivoted to eliminate the stop position after a certain time delay, while the photocell is adapted, in the course of said time delay, to emit a separate signal to the second stop means for dispensing an unused syringe solely on positively sensing a used syringe in said sensing position.

For practical reasons it is preferred furthermore that the collection container constitutes a separate container able to be closed off which is readily mountably and readily dismountably connected to the connecting duct.

In order to avoid undesired manipulation of the automat, for example on the insertion of objects which deviate from the form of a syringe, the automat is characterised in that the intake comprises a combined closing and control arrangement with a positive control mechanism for the controlled transfer of a used syringe from an outwardly uncovered syringe bed outside the automat to a closed discharge position inside the automat.

In this connection it is preferred that the closing and control arrangement comprises a first stationarily arranged part in the form of a guide sleeve and a second part axially displaceably and rotatably mounted relative to the first part in the form of a liner body with associated syringe bed internally in the liner body, the one of the parts being provided with a guide pin, while the second of the parts is provided with a helical guide groove, and that the syringe bed of the liner body is adapted to be positively controlled from an uncovered upwardly opening filling position to a downwardly opening discharge position by means of a positive control between the guide pin and the guide groove, on axially displacing the liner body from a filling position outside the automat to a discharge position within the automat.

In order to ensure that only syringe-like objects are sensed in the automat the latter is characterised in that the connecting duct comprises a guide and control rail extending obliquely downwards having control flaps for the support of opposite parts of the widest portion of a used syringe with the remainder of the syringe projecting downwards below the guide rail and having an intermediate groove for reception of the narrower, downnwardly projecting portion of the used syringe, and that sensing means for sensing a used syringe are arranged on the under side of the control rail at its free ending lower end, for exclusively actuating the narrower, downwardly projecting portion of the syringe.

There is hereby the possibility of allowing imitation insert bodies to be led outside and thereby to be sorted away from the sensing means for sensing the used syringe. This can happen by either allowing the insert body to pass vertically through the guide and control rail upstream in front of the sensing means or by allowing the insert body to be led outside the sensing means on the top of the guide and control rail, and thereby without the possibility of being brought into a path past the sensing means on the under side of the guide and control rail.

Further features of the present invention will be evident from the following description having regard to the accompanying drawings, in which:
Fig. 1 shows in a front view a front plate on an automat according to the invention according to a first embodiment.
Fig. 2 shows an automat according to the invention represented schematically, partially in side view and partially in vertical section.
Fig. 3 shows in side view a collection container which constitutes a separate part of the automat according to Fig. 1.
Fig. 4 shows in side view a section of an automat according to the invention, in a second embodiment.
Fig. 5 and 6 show a control arrangement for the automat according to Fig. 4 in two different working positions, where the control arrangement is shown from above in the one position (Fig. 5) and in the other position (Fig. 6) is shown from below.
Fig. 7 shows the same as in Fig. 6, illustrated in vertical section.
Fig. 8 shows a detail of Fig. 4 illustrated in plan view.
Fig. 9 shows the same detail as shown in Fig. 8, illustrated in end view.

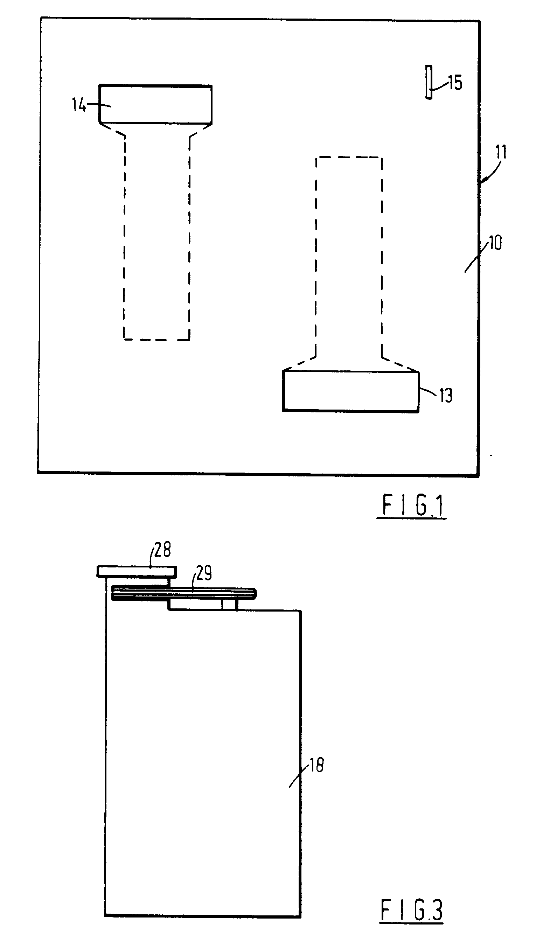

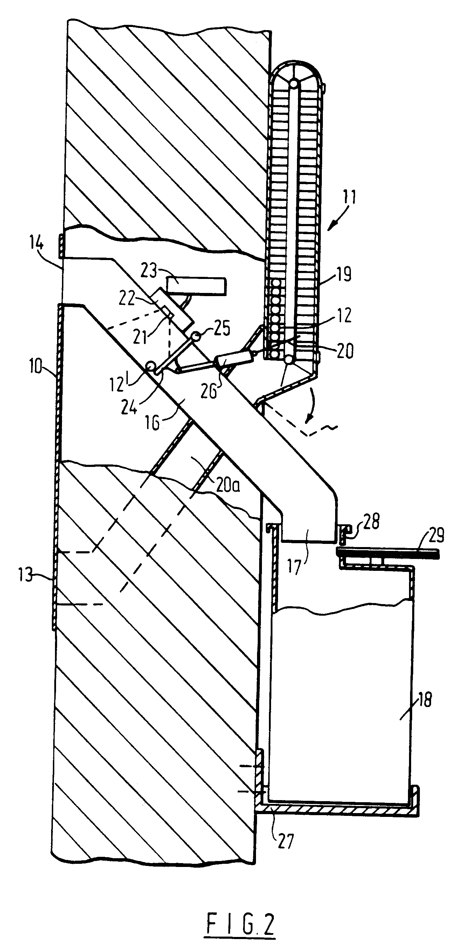

In Fig. 1 and 2 there is shown a front plate 10 for an automat 11 for dispensing unused one-time syringes 12 via an outlet 13 and for receiving used one-time syringes 12' via an intake 14. The automat is adapted to give up an unused one-time syringe 12 when a used one-time syringe 12' is installed. In addition there is shown a coin insert 15 with which an unused one-time syringe can be given up exclusively by the insertion of coins. In the instances such a coin insert is employed in the automat, in addition to the mechanism for dispensing an unused one-time syringe 12 for each insert of a used one-time syringe, there can be employed a coin insert mechanism known per se.

The mechanism for giving up an unused syringe in exchange for a used syringe can as mentioned be used together with the coin insert mechanism, but can also be used independently of the coin insert mechanism.

The automat can be erected in arbitrary, publicly accessible locations or in specific locations which are specially designed for special users.

The automat can also be used at dentists' offices, doctors' offices or other arbitrary locations where syringes are placed and where there is a need for easy access to unused syringes and immediately thereafter used syringes can be effectively disposed of without substantial danger of the transfer of infection via used syringes.

In the illustrated embodiment there extends from the intake 14 on the front side 10 of the automat 11 a connecting duct 16 for used syringes 12' obliquely downwards and inwards into the automat to an outlet 17 to a collection container 18. Just by the side of the insert mechanism for used syringes there is arranged a mechanism for dispensing unused syringes 12. There is shown a paternoster-like storage space 19 arranged just above a dispensing mechanism 20 which is connected to a discharge duct 20a extending obliquely downwards and outwards to the outlet 13 for unused syringes.

In the connecting duct 16 there is arranged a photocell 21 in a recording box 22 which is connected to a comparing arrangement 23. The photocell 21 is arranged just in front and just above a flap 24 which is pivotably mounted about an upper pivotal axis at 25. The flap 24 is controlled by an hydraulically, pneumatically or electrically driven cylinder 26 which with a certain time delay is adapted to rotate the flap 24 after the latter is mechanically actuated by an object which is introduced via the intake 14 of the connecting duct 16. In the course of said time delay the photocell 21 together with the comparing arrangement 23 are adapted to search the inserted object. If the object which is inserted deviates from a one-time syringe the passage is opened by rotation of the flap 24 for the delivery of the object to the collection container 18. If the photocell with associated comparing arrangement establishes that a (used) syringe is received a pulse or a signal is emitted to the dispensing mechanism 20 so that an unused syringe 12 is dispensed from the store to the outlet 13.

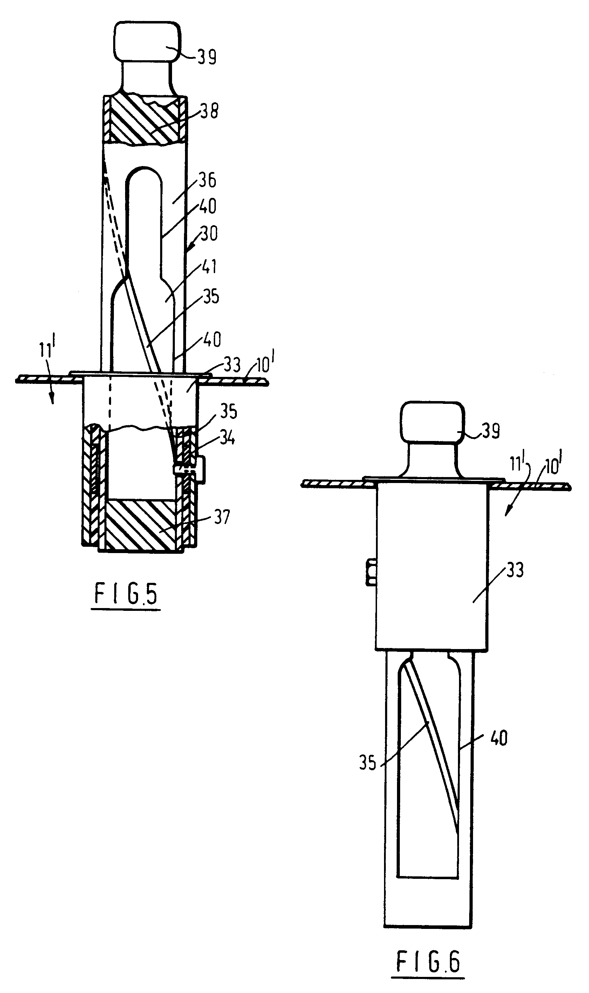

As shown in Fig. 2 the collection container 18 rests on a wall fitting 27 with a container opening 28 flush with the outlet 17 of the connecting duct 16. A laterally displaceable cover 29, which is pushed away from the outlet 17 and the opening 28 in the position as illustrated in Fig. 2 is pushed in the position as illustrated in Fig. 3 into place over the opening 28 for effective closing off of the collection container for transport and storage of the latter.

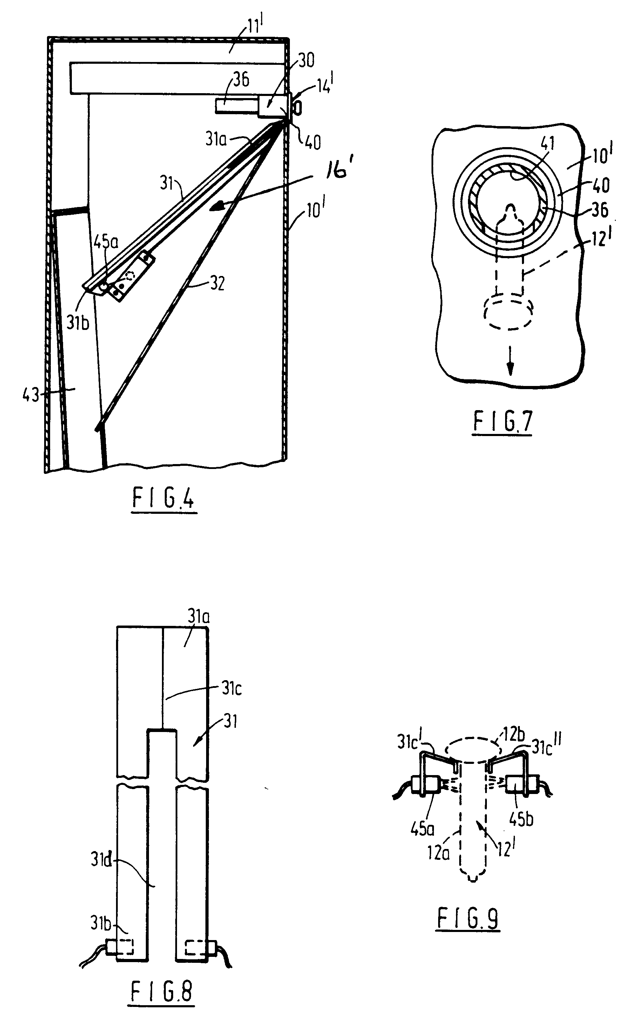

In an alternative embodiment, as shown generally in Fig. 4, the intake 14' is provided with a combined closing and control arrangement 30, such as further illustrated in Fig. 5-7. Further the connecting duct 16' is divided up into a combined guide and sorting rail 31, as is shown in Fig. 8 and 9, and into a bottom guide plate 32 as shown in Fig. 4.

The closing and control arrangement 30 comprises a control sleeve 33 which is stationarily fixed to the automat 11' at its front side 10'. In the sleeve 33 there is fastened a control pin 34 projecting radially inwards which engages a helical line-shaped guide groove 35 in a liner member 36 which is rotatably mounted and axially displaceable in the sleeve 33. On axially displacing the liner member 36 in the sleeve 33 the insert member will be positively adjusted an equivalent angle of rotation relative to the automat, that is to say is rotated an angle of 180° about the longitudinal axis. In this way one can achieve a favorable filling position with the liner member in an extended, upwardly uncovered position and a favorable discharge position with the liner member in a pushed-in position facing downwards. In this manner tampering with the mechanism in an unintentional manner for the release of an unused syringe as a reaction to the insertion of a used syringe can be prevented, the liner member 36 forming a stop arrangement in the automat even after the liner member is partially pushed in towards the discharge position.

The liner member 36 is shown in the form of a pipe piece with a first end piece 37 in the one end and with a second end piece 38 in the other end. The end piece 38, which faces outwardly on the front side of the automat, is rotatably connected on the outwardly facing side to a finger grip or operating grip 39. Between the end pieces 37 and 38 on one side portion of the pipe piece there is formed a cavity 40 which forms an access opening to a bed 41 for the picking up of a used syringe 12', while on the opposite side portion of the pipe piece there is formed said guide groove 35.

In Fig. 5 the liner member 36 is illustrated in an extended outer position relative to the automat, in which the access opening 40 is uncovered for the insertion of a used syringe 12' in the bed 41. In Fig. 6 the liner member 36 is shown in a pushed-in inner position relative to the automat with the liner member rotated 180°. On pushing in the liner member 36 with asociated used syringe 12' from the position illustrated in Fig. 5 to that illustrated in Fig. 6 the liner member 36 is positively rotated an angle of rotation of 180°. By this the used syringe inside the automat is discharged vertically above and just by the upper inner end 31a of the guide and control rail 31. The used syringe thereafter slides obliquely downwards (at an angle of 45-60°) along the guide and control rail 31 towards the lower outer end 31b of the rail where the used syringe is discharged via an approximately vertical guide duct 43 to a collection container not shown further corresponding to the container 18 in Fig. 2.

The guide and control rail 31 has an approximately M-shaped cross-section, such as shown in Fig. 9, that is to say with a V-shaped web portion 31c which forms the guide or control portion itself for a used syringe on the rail 31. In a short upper portion at the upper end of the web portion 31c, that is to say at the inner end 31a of the rail 31, the web portion 31c is undivided and has a continuous V-shaped cross-section, while in the remaining portion of the web portion 31c lying below, including the lower end 31b of the rail 31, there is formed a through, longitudinal groove 31d.

After the used syringe is discharged on the short, undivided upper portion of the rail 31 the syringe is adapted to be conducted downwards along the rail 31 and to be guided automatically to an intended sensing position with certain (narrow) main parts 12a of the syringe pivoted downwards in the groove 31d and with the (further) projected head portion or actuating portion 12b of the syringe resting against adjacent flaps 31c' and 31c'' of the remaining V-shaped middle portion 31. On the under side of the rail 31, at its lower end, there are arranged a pair of photocells or similar sensors 45a, 45b which are only adapted to be actuated by an object which is led in a path past the sensors on the under side of the rail 31.

In this way by the aid of simple means objects which correspond to the shape of a syringe can be sorted out. Other objects, which have to have a shape which deviates substantially from the shape of the syringe, will then - either fall through groove 31d of the rail at the upper or central portion of the rail and be conducted along the other guide rail 32 extending downwards more steeply and via the guide duct 43 to the collection container - or will be conducted endways along the rail 31 and be delivered at its lower end and be conducted in its totality from the upper side of the rail 31 via the guide duct 43 to the collection container. Only the objects, which have certain portions (12a) which project sufficiently far downwards on the under side of the groove 31d at the lower end of the rail 31 and which have remaining portions (12b) which rest against the flaps 31c' and 31c'' which define the groove 31d, will actuate the sensors 45a, 45b and only these objects will be able to release a control signal from the sensors 45a, 45b to the mechanism for giving up an unused syringe as a reaction to the insertion of a used syringe into the automat.

All objects which are emptied into the automat via the intake 14' and the associated liner body 36 are discharged automatically into the collection container, but only the objects which have the form of a syringe are conducted in such a manner that they can be sensed by the sensors 45a, 45b and only these can bring about the automatic release of unused syringes as a reaction to the insertion of a syringe-shaped body.

## Claims

1. Automat (11) for one-time syringes (12,12'), where a number of unused syringes (12) are stored in a storage space (19) in the automat and can be dispensed individually via an outlet (13) in the automat on actuation of an actuating means (21-26), characterised in that the automat (11) comprises a collection chamber (18) for receiving used syringes (12'), and that an intake (14, 14')for used syringes (12') communicates with the collection chamber (18) which is different from and separated from the storage space (19) for unused syringes (12) via a connecting duct (16; 30-33, 36, 43) which includes the actuating means or a special actuating means (21; 45a,45b) for sensing a used syringe and for activating the dispensing of an unused syringe (12) via a separate outlet (13) as a reaction to the insertion of the used syringe (12') in said connecting duct.

2. Automat in accordance with claim 1, characterised in that the connecting duct (16) includes a first stop means (the flap 24), for intermittently shutting off a used syringe (12') in a sensing position until the used syringe is discharged further into the collection container (18), the stop means (24) on actuation by an inserted, used syringe (12') being adapted to activate a second stop means (the cylinder 26) for dispensing an unused syringe (12) from the storage space (19), as a reaction to the actuation of the first stop means (24).

3. Automat in accordance with claim 1 or 2, characterised in that the connecting duct (16) includes a sensing means (photocell 21) for sensing a used syringe (12) in a sensing position in the conecting duct, a positive sensing of a used syringe in the connecting duct releasing a signal for activating a dispensing of an unused syringe (12).

4. Automat in accordancce with the claims 1-3, characterised in that the first stop means comprises a flap (24) with a normal initial position for shutting off an injected used syringe (12') in a sensing position in the connecting duct (16), and that the sensing means comprises a photocell (21) which is arranged directly on or upstream immediately in front of the flap, the flap being adapted to be pivoted to eliminate the stop position after a certain time delay, while the photocell is adapted, in the course of said time delay, to emit a separate signal to the second stop means (26) for the dispensing of an unused syringe (12) solely by positive sensing of a used syringe (12') in said sensing position.

5. Automat in accordance with claim 4, characterised in that the collection container (18) constitutes a separate container able to be closed off which is readily mountably and readily dismountably connected to outlet (17) of the connecting duct (16).

6. Automat in accordance with claim 1, characterised in that the intake (14') comprises a combined closing and control arrangement (30) with a positive control mechanism (33-36) for the controlled transfer of a used syringe (12') from an outwardly uncovered syringe bed (41) outside the automat to a closed discharge position inside the automat.

7. Automat in accordance with claim 6, characterised in that the closing and control arrangement (30) comprises a first stationarily arranged part in the form of a guide sleeve (33) and a second part axially displaceably and rotatably mounted relative to the first part in the form of a liner body (36) with associated syringe bed (41) internally in the liner body, the one of the parts being provided with a guide pin (34), while the second of the parts is provided with a helical guide groove (35), and that the syringe bed (41) of the liner body (36) is adapted to be positively controlled from an uncovered upwardly opening filling position to a downwardly opening discharge position by means of a positive control between the guide pin (34) and the guide groove (35), on axially displacing the liner body from a filling position outside the automat to a discharge position within the automat.

8. Automat in accordance with claim 1, characterised in that the connecting duct (16') comprises a guide and control rail (31) extending obliquely downwards having control flaps (31c', 31c'') for the support of opposite parts of the widest portion (12b) of a used syringe (12') with the remainder of the syringe projecting downwards below the guide rail and having an intermediate groove (31d) for the reception of the narrower, downwardly projecting portion (12a) of the used syringe, and that sensing means (45a, 45b) for sensing a used syringe are arranged on the under side of the control rail (31) at its free ending lower end, for exclusively actuating the narrower, downwardly projecting portion of the syringe.

## Patentansprüche

1. Automat (11) für Wegwerfspritzen (12, 12'), wo eine Anzahl unbenutzter Spritzen (12) in einem im Automaten vorhandenen Vorratsraum (19) untergebracht sind und bei Betätigung eines Betätigungsmittels (21-26) durch einen Auslass (13) des Automaten einzeln ausgegeben werden können, dadurch **gekennzeichnet**, dass der Automat (11) eine Sammelkammer (18) zur Aufnahme benutzter Spritzen (12') umfasst, und dass ein für benutzte Spritzen (12') vorgesehener Einlass (14, 14') mit der Sammelkammer (18) in Verbindung steht, welche sich vom Vorratsraum (19) für unbenutzte Spritzen (12) unterscheidet und durch einen Verbindungskanal (16; 30-33, 36, 43) davon getrennt ist, welcher Kanal das Betätigungsmittel oder ein spezielles Betätigungsmittel (21; 45a, 45b) aufnimmt, das eine benutzte Spritze abtastet und als Reaktion auf das Einführen der benutzten Spritze (12') in den genannten Verbindungskanal die Ausgabe einer unbenutzten Spritze (12) durch einen getrennten Auslass (13) auslöst.

2. Automat nach Anspruch 1, dadurch **gekennzeichnet**, dass der Verbindungskanal (16) ein erstes Sperrglied (den Lappen 24) enthält, um in einer Abtastposition eine benutzte Spritze (12') intermittierend abzusperren, bis diese weiter in die Sammelkammer (18) hineingefördert wird, wobei das von einer eingeführten, benutzten Spritze (12') betätigte Sperrglied (24) ein zweites Sperrglied (den Zylinder 26) betätigt, das als Reaktion auf die Betätigung des ersten Sperrglieds (24) eine unbenutzte Spritze (12) vom Vorratsraum (19) ausgibt.

3. Automat nach Anspruch 1 oder 2, dadurch **gekennzeichnet**, dass der Verbindungskanal (16) ein Abtastglied (eine Photozelle 21) enthält, welches in einer Abtastposition im Verbindungskanal eine benutzte Spritze (12) abtastet, wobei ein positives Abtasten einer benutzten Spritze im Verbindungskanal ein Signal abgibt, das die Ausgabe einer unbenutzten Spritze (12) auslöst.

4. Automat nach den Ansprüchen 1-3, dadurch **gekennzeichnet**, dass das erste Sperrglied einen Lappen (24) mit einer normalen Ausgangsposition zum Absperren einer benutzten, injizierten Spritze (12') in einer Abtastposition im Verbindungskanal (16) aufweist, und dass das Abtastglied eine Photozelle (21) hat, die direkt am Lappen oder stromabwärts unmittelbar vor dem Lappen angebracht ist, welcher schwenkbar angeordnet ist, um nach einer gewissen Verzögerung die Sperrposition aufzuheben, während die Photozelle im Laufe der genannten Verzögerung dem zweiten Sperrglied (26) ein Sondersignal zur Ausgabe einer unbenutzten Spritze (12) abgibt, lediglich wenn eine benutzte Spritze (12') in der genannten Abtastposition positiv abgetastet wird.

5. Automat nach Anspruch 4, dadurch **gekennzeichnet**, dass die Sammelkammer (18) ein separater, abschliessbarer Behälter ist, der mit dem Auslass (17) des Verbindungskanals (16) leicht aufsetzbar und abnehmbar verbunden ist.

6. Automat nach Anspruch 1, dadurch **gekennzeichnet**, dass der Einlass (14') eine kombinierte, mit einer zwangsläufigen Steuerung (33-36) versehene Schliess- und Steuervorrichtung (30) aufweist, die die Überführung einer benutzten Spritze (12') von einem nach aussen nicht abgedeckten Spritzenbett (41) ausserhalb des Automaten zu einer geschlossenen Ausgabeposition im Automaten steuert.

7. Automat nach Anspruch 6, dadurch **gekennzeichnet**, dass die Schliess- und Steuervorrichtung (30) einen ersten, ortsfesten Teil in Form einer Führungshülse (33) und einen zweiten Teil in Form eines Einlagekörpers (36) mit einem zugehörigen Spritzenbett (41) im Inneren des Einlagekörpers umfasst, welcher zweiter Teil im Verhältnis zum ersten Teil axial versetzbar und drehbar angebracht ist, wobei einer dieser Teile mit einem Führungsstift (34) versehen ist, während der andere mit einer schraubenförmigen Führungsnut (35) ausgebildet ist, und dass das Spritzenbett (41) des Einlagekörpers (36) durch eine zwangsläufige Steuerung zwischen dem Führungsstift (34) und der Führungsnut (35) zwangsläufig aus einer nicht abgedeckten, nach oben hin offenen Füllposition in eine nach unten hin offene Ausgabeposition gesteuert wird, wenn der Einlagekörper von einer Füllposition ausserhalb des Automaten zu einer Ausgabeposition im Automaten axial versetzt wird.

8. Automat nach Anspruch 1, dadurch **gekennzeichnet**, dass der Verbindungskanal (16') eine schräg nach unten sich erstreckende, mit Steuerlappen (31c', 31c'') versehene Führungs- und Steuerschiene (31) aufweist, um entgegengesetzte Teile des breitesten Abschnitts (12b) einer benutzten Spritze (12') abzustützen, während sich der Rest der Spritze unter der Führungsschiene nach unten erstreckt, wobei die Schiene (31) eine Zwischennut (31d) zur Aufnahme des schmaleren, nach unten sich erstreckenden Abschnitts (12a) der benutzten Spritze aufweist, und dass die zum Abtasten einer benutzten Spritze vorgesehenen Abtastglieder (45a, 45b) an der Unterseite der Führungsschiene (31) an deren freiem unterem Ende angeordnet sind, um ausschliesslich den schmaleren, nach unten sich erstreckenden Abschnitt der Spritze zu betätigen.

## Revendications

1. Appareil automatique (11) pour seringues à usage unique (12, 12') dans lequel un certain nombre de seringues non utilisées (12) sont stockées dans un espace de stockage (19) à l'intérieur de l'appareil automatique et peuvent être distribuées individuellement par une sortie (13) de l'appareil automatique par actionnement d'un dispositif d'actionnement (21-26), caractérisé en ce que l'appareil automatique (11) comprend une chambre collectrice (18) destinée à recevoir des seringues utilisées (12'), et en ce qu'une entrée (14,14') pour les seringues utilisées (12') communique avec la chambre collectrice (18), qui est différente de l'espace de stockage (19) pour les seringues non utilisées (12) et qui en est séparée, par l'intermédiaire d'une conduite de liaison (16 ; 30-33, 36, 43) qui comprend le dispositif d'actionnement ou un dispositif d'actionnement spécial (21 ; 45a, 45b) pour détecter une seringue utilisée et pour activer la distribution d'une seringue non utilisée (12) par une sortie séparée (13) en réaction à l'insertion de la seringue utilisée (12') dans ladite conduite de liaison.

2. Appareil automatique selon la revendication 1, caractérisé en ce que la conduite de liaison (16) comprend un premier dispositif d'arrêt (volet 24) pour arrêter de manière intermittente une seringue utilisée (12') dans une position de détection jusqu'à ce que la seringue utilisée soit évacuée encore dans le réservoir collecteur (18), le dispositif d'arrêt (24) étant conçu, par actionnement par une seringue utilisée (12') insérée, pour activer un second dispositif d'arrêt (cylindre 26) pour distribuer une seringue non utilisée (12) depuis l'espace de stockage (19) en réaction à l'actionnement du premier dispositif d'arrêt (24).

3. Appareil automatique selon la revendication 1 ou 2, caractérisé en ce que la conduite de liaison (16) comprend un dispositif détecteur (cellule photoélectrique 21) pour détecter une seringue utilisée (12') dans une position de détection dans la conduite de liaison, une détection positive d'une seringue utilisée dans la conduite de liaison produisant un signal pour activer la distribution d'une seringue non utilisée (12).

4. Appareil automatique selon les revendications 1 à 3, caractérisé en ce que le premier dispositif d'arrêt comprend un volet (24) présentant une position initiale normale pour arrêter une seringue utilisée (12') injectée dans une position de détection dans la conduite de liaison (16), et en ce que le dispositif détecteur comprend une cellule photoélectrique (21) qui est disposée directement sur le volet ou en amont immédiatement devant le volet, le volet étant conçu pour pivoter afin d'éliminer la position d'arrêt au bout d'un certain délai, tandis que la cellule photoélectrique est conçue, au cours dudit délai, pour envoyer un signal séparé au second dispositif d'arrêt (26) pour la distribution d'une seringue non utilisée (12) uniquement par détection positive d'une seringue utilisée (12') dans ladite position de détection.

5. Appareil automatique selon la revendication 4, caractérisé en ce que le récipient collecteur (18) constitue un récipient séparé qui peut être fermé et qui est relié à la sortie (17) de la conduite de liaison (16) de manière facilement montable et facilement démontable.

6. Appareil automatique selon la revendication 1, caractérisé en ce que l'entrée (14') comprend un agencement de fermeture et de commande combinées (30) avec un mécanisme de commande positive (33-36) pour le transfert commandé d'une seringue utilisée (12') depuis un logement de seringue (41) non recouvert à l'extérieur, situé à l'extérieur de l'appareil automatique, jusque dans une position d'évacuation fermée à l'intérieur de l'appareil automatique.

7. Appareil automatique selon la revendication 6, caractérisé en ce que l'agencement de fermeture et de commande (30) comprend une première partie fixe sous forme d'un manchon de guidage (33) et une seconde partie déplaçable axialement et montée de manière à pouvoir tourner par rapport à la première partie sous forme d'un corps de boîtier (36), le logement de seringue (41) associé étant situé à l'intérieur du corps de boîtier, l'une des parties étant munie d'une broche de guidage (34) tandis que la seconde des parties est munie d'une rainure de guidage hélicoïdale (35), et en ce que le logement de seringue (41) du corps de boîtier (36) est conçu pour être commandé positivement depuis une position de remplissage non recouverte, ouverte vers le haut, jusque dans une position d'évacuation ouverte vers le bas au moyen d'une commande positive entre la broche de guidage (34) et la rainure de guidage (35), lors du déplacement axial du corps de boîtier depuis une position de remplissage à l'extérieur de l'appareil automatique jusque dans une position d'évacuation à l'intérieur de l'appareil automatique.

8. Appareil automatique selon la revendication 1, caractérisé en ce que la conduite de liaison (16') comprend un rail de guidage et de commande (31) qui s'étend obliquement vers le bas, qui comporte des bandes de commande (31c', 31c'') pour soutenir les parties opposées de la partie la plus large (12b) d'une seringue utilisée (12'), le reste de la seringue faisant saillie vers le bas au-dessous du rail de guidage, et qui comporte une rainure intermédiaire (31d) destinée à recevoir la partie plus étroite (12a), qui fait saillie vers le bas, de la seringue utilisée, et en ce que des dispositifs détecteurs (45a, 45b) pour détecter une seringue utilisée sont disposés sur le côté inférieur du rail de commande (31) au niveau de son extrémité inférieure qui se termine librement, pour actionner exclusivement la partie plus étroite de la seringue qui fait saillie vers le bas.
